(19) Europäisches Patentamt
European Patent Office
Office européen des brevets

(11) Numéro de publication: **0 295 161 B1**

(12) **FASCICULE DE BREVET EUROPEEN**

(45) Date de publication de fascicule du brevet: **11.11.92**

(51) Int. Cl.[5]: **C07D 471/04**, A61K 31/435, //(C07D471/04,235:00,221:00)

(21) Numéro de dépôt: **88401186.7**

(22) Date de dépôt: **17.05.88**

(54) **Dérivés d'acylaminométhyl-3 tétrahydro-5,6,7,8 imidazo[1,2-a] pyridines, leur préparation et leur application en thérapeutique.**

(30) Priorité: **21.05.87 FR 8707125**

(43) Date de publication de la demande:
**14.12.88 Bulletin 88/50**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:
**EP-A- 0 172 096**
**FR-A- 2 593 817**

(73) Titulaire: **SYNTHELABO**
**58 rue de la Glacière**
**F-75013 Paris(FR)**

(72) Inventeur: **George, Pascal**
**39, rue Henri de Vilmorin**
**F-94400 Vitry sur Seine(FR)**
Inventeur: **Giron, Claudie**
**1 Parvis de la Bièvre Appt. 76**
**F-92160 Antony(FR)**

(74) Mandataire: **Ludwig, Jacques et al**
**SYNTHELABO, Service Brevets, 22, avenue Galilée, B.P. 72**
**F-92352 Le Plessis Robinson Cédex(FR)**

EP 0 295 161 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés d'acylaminométhyl-3 tétrahydro-5,6,7,8 imidazo[1,2-a]-pyridines, leur préparation et leur application en thérapeutique.

Les composés de l'invention répondent à la formule générale (I)

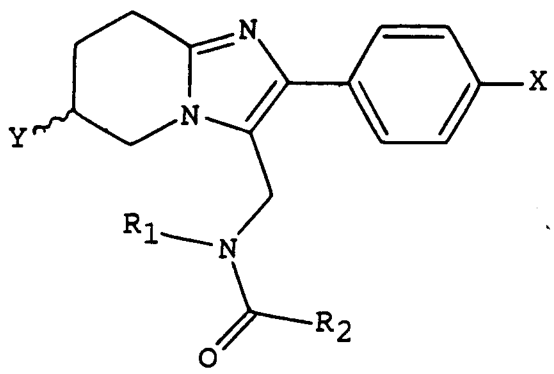

(I)

dans laquelle

X représente un atome d'hydrogène ou d'halogène, un groupe alcoxy en $C_1$-$C_4$ ou un groupe alkyle en $C_1$-$C_6$,

Y représente un atome d'hydrogène ou un groupe méthyle,

$R_1$ représente un groupe alkyle en $C_1$-$C_4$, et

$R_2$ représente un groupe alkyle en $C_1$-$C_6$.

Les composés de l'invention peuvent se présenter à l'état de bases libres ou de sels d'addition à des acides acceptables en pharmacologie. Lorsque Y représente un groupe méthyle, l'atome de carbone qui le porte est asymétrique. Les composés de l'invention peuvent donc encore se présenter sous forme d'énantiomères purs, ou de leurs mélanges.

Les composés préférés sont ceux dans la formule (I) desquels X représente un atome de chlore ou un groupe méthyle, $R_1$ représente un groupe méthyle et $R_2$ représente un groupe n-propyle ou isobutyle.

Conformément à l'invention on peut préparer les composés de formule (I) par hydrogénation de leurs analogues insaturés aux positions 5,6, 7 et 8. Ces derniers sont décrits dans le brevet des Etats-Unis d'Amérique n° 4650796.

L'hydrogénation est de type catalytique sous pression, c'est-à-dire qu'on l'effectue par exemple en présence de palladium ou de rhodium absorbé sur un support tel que le charbon ou l'alumine. Le composé de départ est utilisé sous forme de base ou de sel, par exemple le chlorhydrate, dans un solvant tel qu'un alcool aliphatique, par exemple le méthanol ou l'éthanol, ou dans un solvant acide tel que l'acide acétique.

Les exemples suivants illustrent la préparation de quelques composés selon l'invention. La microanalyse et les spectres IR et RMN confirment les structures des produits obtenus.

### Exemple 1.

Chlorhydrate de N-[[(méthyl-4 phényl)-2 méthyl-6 tétrahydro-5,6,7,8 imidazo[1,2-a]pyridinyl-3]méthyl] N,3-diméthyl-butanamide.

On dissout 1 g (2,86 mmoles) de N-[[(méthyl-4 phényl)-2 méthyl-6 imidazo[1,2-a]pyridinyl-3]méthyl]N,3-diméthyl-butanamide dans 29 ml d'alcool isopropylique chlorhydrique 0,1N, y ajoute 50 ml d'alcool isopropylique et 0,5 g de charbon palladié à 10 %, et on hydrogène sous environ 0,35 MPa (50 PSI) pendant 8 h.

On filtre le catalyseur, on chasse le solvant sous pression réduite, et on traite le résidu à l'éther. On obtient 0,8 g de produit attendu sous forme de chlorhydrate.

F = 195-196 °C.

### Exemple 2.

Chlorhydrate de N-[[(hexyl-4 phényl)-2 méthyl-6 tétrahydro-5,6,7,8 imidazo[1,2-a]pyridinyl-3]méthyl] N,3-diméthyl-butanamide.

On dissout 3,53 g (8,4 mmoles) de chlorhydrate de N-[[(hexyl-4 phényl)-2 méthyl-6 imidazo[1,2-a]-

pyridinyl-3]méthyl]N,3-diméthyl-butanamide dans 64 ml d'éthanol à 95 % contenant 1,5 g de charbon palladié à 10 %. On hydrogène sous pression d'environ 0,35 MPa (50 PSI) jusqu'à absorption de 2 moles d'hydrogène. On élimine le catalyseur par filtration et on évapore le solvant sous pression réduite. On dissout le résidu d'évaporation dans du dichlorométhane et on élimine l'insoluble par filtration. On concentre le filtrat sous pression réduite et on lave le résidu à l'éther. On obtient 3,03 g de solide blanc.
F = 161-162 °C.

Exemple 3.

Chlorhydrate de N-[[(chloro-4 phényl-2 méthyl-6 tétrahydro-5,6,7,8 imidazo[1,2-a]pyridinyl-3]méthyl] N,3-diméthyl-butanamide.

On dissout 3 g (8,1 mmole) de chlorhydrate de N-[[(chloro-4 phényl]-2 méthyl-6 imidazo[1,2-a]pyridinyl-3]méthyl] N,3-diméthyl-butanamide dans 200 ml d'acide acétique, y ajoute 0,7 g de rhodium sur alumine à 5% et on hydrogène sous une pression d'environ 0,35 MPa (50 PSI) jusqu'à fin d'absorption. On élimine le catalyseur par filtration, on évapore le solvant sous pression réduite. On reprend le résidu avec du dichlorométhane, on lave la solution à l'eau bicarbonatée, on décante la phase organique et on la sèche sur sulfate de magnésium. Après filtration et évaporation du solvant, on reprend le résidu solide à l'éther, puis on le purifie par chromatographie sur colonne de silice. On en prépare le chlorhydrate dans l'alcool isopropylique chlorhydrique 0,1 N. On obtient 1,55 g de produit attendu.
F = 197-198 °C.

Le tableau ci-après illustre les structures et propriétés physiques de quelques composés selon l'invention.

Tableau

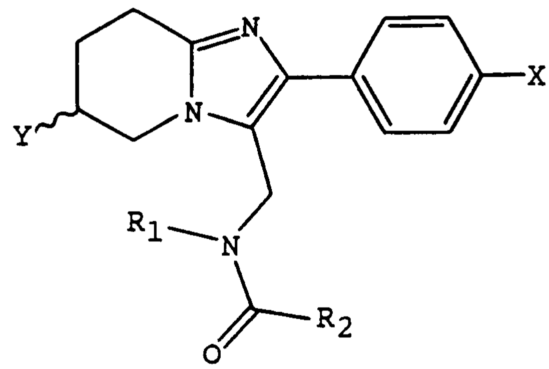

(I)

| N° | X | Y | $R_1$ | $R_2$ | F(°C) |
|---|---|---|---|---|---|
| 1 | Cl | H | $CH_3$ | $i\text{-}C_4H_9$ | 193-195* |
| 2 | $n\text{-}C_3H_7$ | H | $CH_3$ | $i\text{-}C_4H_9$ | 197-199* |
| 3 | Cl | $CH_3$ | $CH_3$ | $nC_3H_7$ | 194-196* |
| 4 | Cl | $CH_3$ | $CH_3$ | $i\text{-}C_4H_9$ | 197-198* |
| 5 | $CH_3$ | $CH_3$ | $CH_3$ | $i\text{-}C_4H_9$ | 195-196* |
| 6 | $C_2H_5$ | $CH_3$ | $CH_3$ | $i\text{-}C_4H_9$ | 196-198* |
| 7 | $n\text{-}C_3H_7$ | $CH_3$ | $CH_3$ | $i\text{-}C_4H_9$ | 184-186* |
| 8 | $n\text{-}C_6H_{13}$ | $CH_3$ | $CH_3$ | $i\text{-}C_4H_9$ | 161-163* |
| 9 | $OCH_3$ | H | $CH_3$ | $i\text{-}C_4H_9$ | 173-175* |

* : chlorhydrate

Les composés de l'invention ont fait l'objet d'essais pharmacologiques qui ont mis en évidence leur intérêt comme substances à activités thérapeutiques.

Toxicité aigüe.

Les $DL_{50}$ (doses létales à 50 %) sont supérieures ou égales à 300 mg/kg, chez la souris, par voie orale.

Antagonisme vis-à-vis des convulsions cloniques induites par le Cardiazol® chez la souris.

L'essai est inspiré du protocole décrit par Goodman et al., J. Pharm. Exp. Ther., (1953), 08, 168-176. Les souris reçoivent les produits à tester, ou le solvant seul, par voie intrapéritonéale, 30 minutes (voie intrapéritonéale) ou 60 minutes (voie orale avant l'injection de 35 mg/kg de Cardiazol® par voie intraveineuse. Les animaux sont ensuite observés pendant une heure et, pour chaque lot, le pourcentage de souris présentant des convulsions cloniques est noté (100 % de convulsions cloniques et 10 à 20 % de convulsions toniques chez les animaux de contrôle).

Pour chaque dose, on calcule le pourcentage de protection par rapport aux animaux de contrôle, ce qui permet de déterminer graphiquement la $DA_{50}$, dose qui protège 50 % des animaux vis-à-vis des effets convulsivants du Cardiazol®.

Les $DA_{50}$ des composés de l'invention se situent entre 0,1 et 10 mg/kg par la voie intrapéritonéale et entre 1 et 100 mg/kg par la voie orale.

Action sur l'électrocorticogramme du rat curarisé ventilé. L'activité sédative ou hypnotique des composés a été déterminée par l'observation de leur action sur l'électrocorticogramme du rat selon la méthode décrite par H. Depoortere, Rev. E.E.G. Neurophysiol., 10, 3, 207-214 (1980) et par H. Depoortere et M. Decobert, J. Pharmacol. (Paris), 14, 2, 195-265 (1983).

Les produits à étudier ont été administrés par voie intrapéritonéale aux doses croissantes de 1 à 30 mg/kg. Ils induisent des tracés de sommeil à partir de doses allant de 0,1 à 3 mg/kg.

Effets sur la durée du "sommeil" induit par l'hydroxy-4 butyrate de sodium.

Cette action a été déterminée par l'influence des composés sur la durée du "sommeil" induit par l'hydroxy-4 butyrate de sodium chez le rat curarisé.

Les animaux utilisés sont des rats mâles de souche Charles River de 200 ± 20 g. Les animaux, curarisés par l'alloférine à raison de 1 mg/kg par voie i.p., sont placés sous respiration artificielle à l'aide d'un masque appliqué sur le museau (fréquence respiratoire = 50/minute ; volume respiratoire = 14ml).

L'oesophage est préalablement ligaturé afin d'éviter l'entrée de l'air dans l'estomac.

Des électrodes corticales front-pariétales et occipitales permettent l'enregistrement de l'activité électrocorticographique sur un polygraphe Grass modèle 79 P à la vitesse de 6 mm/sec.

La préparation de l'animal est effectuée sous anesthésie locale (xylocaïne à 2%). Les rats sont maintenus tout au long de l'expérience à température constante (37,5 ° C). Dix minutes après la fin de la préparation du rat, une dose de 200 mg/kg d'hydroxy-4 butyrate de sodium est injectée par voie intraveineuse au niveau de la queue.

Une dose de 10 mg/kg du composé à étudier est administrée par voie intrapéritonéale 3 minutes après l'administration de l'hydroxy-4 butyrate de sodium.

L'évaluation des tracés s'effectue par périodes de 15 minutes durant 75 minutes après l'injection d'hydroxy-4 butyrate de sodium. Durant cette période d'analyse, la durée totale du "sommeil" est déterminée. Une série de 15 témoins permet de préciser la durée du "sommeil" induit par l'hydroxy-4 butyrate de sodium.

L'analyse statistique des résultats est réalisée à l'aide du test "U" de Mann-Whitney.

On constate ainsi que certains composés réduisent les effets du hydroxy-4 butyrate de sodium (jusqu'à 23 % de diminution de la durée du sommeil à la dose de 1 mg/kg), tandis que d'autres potentialisent ces effets (jusqu'à 38 % d'augmentation à la dose de 10 mg/kg). On constate également que les effets peuvent être opposés selon que les composés sont administrés à doses fortes ou à doses faibles.

Les résultats de ces différents tests montrent que les composés de l'invention possèdent des propriétés anxiolytiques, inductrices de sommeil, hypnotiques et anticonvulsivantes ; ils sont donc utiles pour le traitement des états d'anxiété, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale, en gériatrie, ainsi que pour le traitement des absences dues à des traumatismes crâniens et pour le traitement des encéphalopathies métaboliques.

Par ailleurs, en raison de leur affinité pour les récepteurs benzodiazépiniques périphériques, les composés de l'invention sont indiqués également comme agents immunomodulateurs, antithrombotiques, antitumoraux et antagonistes du facteur d'activation des plaquettes (PAF).

Les composés de l'invention peuvent être présentés sous toute forme appropriée pour l'administration par voie orale ou parentérale, par exemple sous forme de comprimés, de dragées, de gélules, de solutions buvables ou injectables, etc, en association avec tout excipient approprié.

La posologie quotidienne peut aller de 0,1 à 100 mg.

**Revendications**

**Revendications pour les Etats contractants suivants : AT, BE, CH, DE, FR, GB, IT, LI, LU, NL, SE**

1. Composés, sous forme d'énantiomères purs ou de mélanges d'énantiomères, répondant à la formule générale (I)

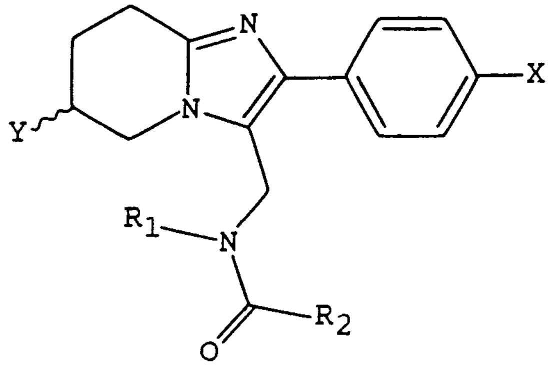

(I)

dans laquelle
X représente un atome d'hydrogène ou d'halogène, un groupe alcoxy en $C_1$-$C_4$ ou un groupe alkyle en $C_1$-$C_6$,
Y représente un atome d'hydrogène ou un groupe méthyle,
$R_1$ représente un groupe alkyle en $C_1$-$C_4$, et
$R_2$ représente un groupe alkyle en $C_1$-$C_6$,
ainsi que leurs sels d'addition à des acides acceptables en pharmacologie.

2. Composés selon la revendication 1, caractérisés en ce que X représente un atome de chlore ou un groupe méthyle, $R_1$ représente un groupe méthyle et $R_2$ représente un groupe n-propyle ou isobutyle.

3. Procédé de préparation d'un composé selon la revendication 1, caractérisé en ce qu'on fait subir une hydrogénation catalytique sous pression à l'analogue insaturé aux positions 5, 6, 7 et 8 d'un composé de formule (I).

4. Composition pharmaceutique, caractérisée en ce qu'elle contient un composé selon la revendication 1, en association avec un excipient.

**Revendication pour les Etats contractants suivants : ES, GR**

1. Procédé de préparation de composés répondant à la formule générale (I)

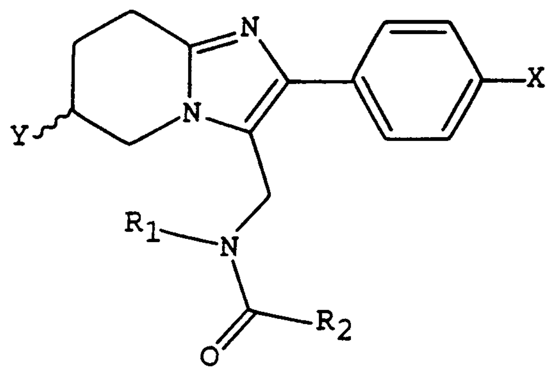

(I)

dans laquelle
X représente un atome d'hydrogène ou d'halogène, un groupe alcoxy en $C_1$-$C_4$ ou un groupe alkyle en $C_1$-$C_6$,

Y représente un atome d'hydrogène ou un groupe méthyle,
$R_1$ représente un groupe alkyle en $C_1$-$C_4$, et
$R_2$ représente un groupe alkyle en $C_1$-$C_6$,
procédé caractérisé en ce qu'on fait subir une hydrogénation catalytique sous pression à l'analogue insaturé aux positions 5, 6, 7 et 8 d'un composé de formule (I).

**Claims**

**Claims for the following Contracting States : AT, BE, CH, DE, FR, GB, IT, LI, LU, NL, SE**

1. Compounds, in the form of pure enantiomers or mixtures of enantiomers, corresponding to the general formula (I)

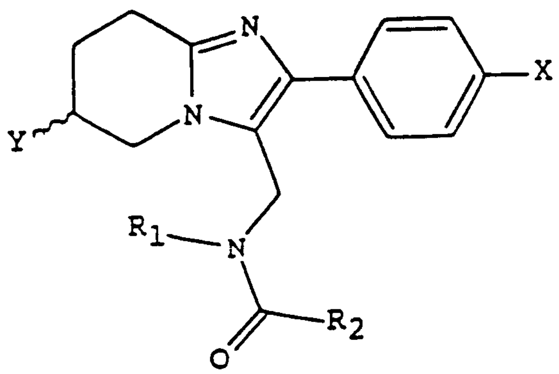

(I)

in which
X denotes a hydrogen or halogen atom, a $C_1$-$C_4$ alkoxy group or a $C_1$-$C_6$ alkyl group,
Y denotes a hydrogen atom or a methyl group,
$R_1$ denotes a $C_1$-$C_4$ alkyl group, and
$R_2$ denotes a $C_1$-$C_6$ alkyl group,
as well as their addition salts with pharmacologically acceptable acids.

2. Compounds according to Claim 1, characterised in that X denotes a chlorine atom or a methyl group, $R_1$ denotes a methyl group and $R_2$ denotes an n-propyl or isobutyl group.

3. Process for preparing a compound according to Claim 1, characterised in that the analogue of a compound of formula (I) which is unsaturated at the 5-, 6-, 7- and 8-positions is subjected to a catalytic hydrogenation under pressure.

4. Pharmaceutical composition, characterised in that it contains a compound according to Claim 1, in combination with an excipient.

**Claim for the following Contracting States : ES, GR**

1. Process for preparing compounds corresponding to the general formula (I)

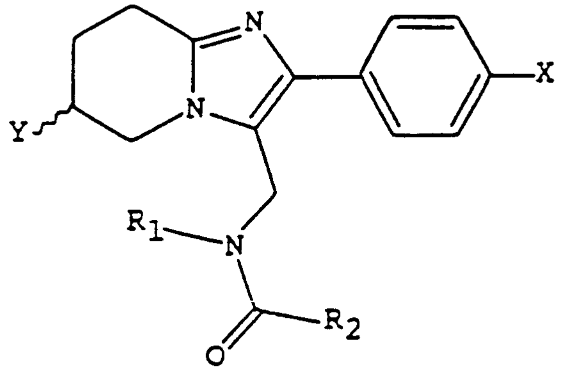

(I)

in which
X denotes a hydrogen or halogen atom, a $C_1$-$C_4$ alkoxy group or a $C_1$-$C_6$ alkyl group,
Y denotes a hydrogen atom or a methyl group,
$R_1$ denotes a $C_1$-$C_4$ alkyl group, and
$R_2$ denotes a $C_1$-$C_6$ alkyl group,
a process which is characterised in that the analogue of a compound of formula (I) which is unsaturated at the 5-, 6-, 7- and 8-positions is subjected to a catalytic hydrogenation under pressure.

**Patentansprüche**
**Patentansprüche für folgende Vertragsstaaten : AT, BE, CH, DE, FR, GB, IT, LI, LU, NL, SE**

1. Verbindungen in Form der reinen Enantiomeren oder von Enantiomerenmischungen der allgemeinen Formel (I)

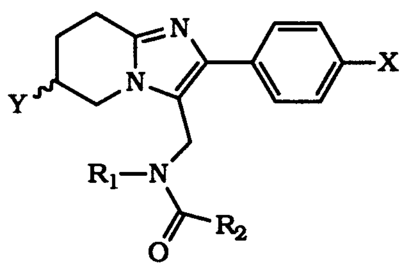

(I)

in der
X ein Wasserstoffatom, ein Halogenatom, eine $C_1$-$C_4$-Alkoxygruppe oder eine $C_1$-$C_6$-Alkylgruppe,
Y ein Wasserstoffatom oder eine Methylgruppe,
$R_1$ eine $C_1$-$C_4$-Alkylgruppe und
$R_2$ eine $C_1$-$C_6$-Alkylgruppe
bedeuten,
sowie deren Additionssalze mit pharmakologisch annehmbaren Säuren.

2. Verbindungen nach Anspruch 1, **dadurch gekennzeichnet,** daß X ein Chloratom oder eine Methylgruppe, $R_1$ eine Methylgruppe und $R_2$ eine n-Propyl- oder Isobutylgruppe bedeuten.

3. Verfahren zur Herstellung einer Verbindung nach Anspruch 1, **dadurch gekennzeichnet,** daß man eine analoge Verbindung der Formel (I), die in den Positionen 5, 6, 7 und 8 ungesättigt ist, einer katalytischen Hydrierung unter Druck unterwirft.

4. Pharmazeutische Zubereitung, **dadurch gekennzeichnet,** daß sie eine Verbindung nach Anspruch 1 in Kombination mit einem Trägermaterial enthält.

**Patentanspruch für folgende Vertragsstaaten : ES, GR**

**1.** Verfahren zur Herstellung der Verbindungen der allgemeinen Formel (I)

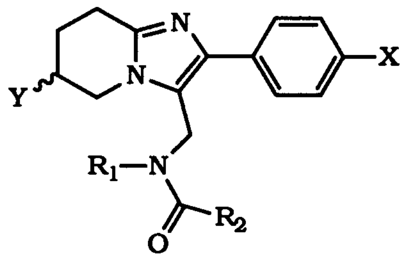

(I)

in der
X ein Wasserstoffatom, ein Halogenatom, eine $C_1$-$C_4$-Alkoxygruppe oder eine $C_1$-$C_6$-Alkylgruppe,
Y ein Wasserstoffatom oder eine Methylgruppe,
$R_1$ eine $C_1$-$C_4$-Alkylgruppe und
$R_2$ eine $C_1$-$C_6$-Alkylgruppe
bedeuten,
**dadurch gekennzeichnet,** daß man eine analoge Verbindung der allgemeinen Formel (I), die in den Positionen 5, 6, 7 und 8 ungesättigt ist, einer katalytischen Hydrierung unter Druck unterwirft.